# EUROPEAN PATENT APPLICATION

(11) **EP 2 255 762 A1**
(43) Date of publication of application: **01.12.2010**
(21) Application number: 09714745.8
(22) Date of filing: 27.02.2009
(51) Int. Cl.: A61F 13/15, A61F 13/472, A61F 13/53, A61F 13/551

(54) **ABSORPTIVE ARTICLE**

(30) Priority: 29.02.2008 JP 2008051073; 31.03.2008 JP 2008093283; 12.02.2009 JP 2009030324
(71) Applicant: Unicharm Corporation, Ehime 799-0111 (JP)
(72) Inventor: The designation of the inventor has not yet been filed
(74) Representative: Kensett, John Hinton
(86) International application number: PCT/JP2009/053714
(87) International publication number: WO 2009/107791

(57) **Abstract**

A sanitary napkin (101) has a liquid-permeable top sheet (102), a liquid-impermeable back sheet (103), and an absorber (104) mounted between the top sheet (102) and the back sheet (103) and capable of absorbing bodily fluid. The sanitary napkin (101) also has insertion holes (107) provided on opposite sides in the lateral direction (W) of the sanitary napkin (101) so as to extend in the longitudinal direction (L) of the sanitary napkin (101), and long members (106) are inserted through the insides of the insertion holes (107). Each of the insertion holes (107) has an opening (108) located on the front end side (Fr) and also has a fixing portion (112) located on the rear end side (Ba). The long members (106) each have a long portion (111) inserted through the inside of the insertion holes (107) and also have a draw-out portion (109) led out from the openings (108) to the outside of the insertion holes (107). The rear ends of the long portion (111) are fixed to the fixing portion (112).

## Description

### [Technical Field]

The present invention relates to an absorptive article used for disposable diaper, sanitary napkin, pantyliner, incontinence pad and so forth.

The present invention relates also to an absorptive article such as sanitary napkin.

### [Background Art]

As a conventional absorptive article, Patent Literature 1 discloses a sanitary napkin having a strip. The strip is provided between a top sheet and a back sheet holding an absorber in between. One end of the strip is exposed to form a tab, whereas the other end of which is fixed between the top sheet and the back sheet.

The user holds the used sanitary napkin by one hand, and pinch and pull a tab of the strip with the other hand. The napkin as a whole is folded like a bellows, as the strip exposes out from the napkin. The napkin is kept in the bellows form, while being finally wrapped therearound by the strip. The napkin is discarded in this state.
Patent Literature 1: JP Patent Application Publication No. 2007-202790

### [Disclosure of the Invention]

The conventional sanitary napkin has, however, a hygienic problem still unsolved in that the user's hand may come into contact with the fouled top sheet, when the user fold the used napkin for disposal.

It is therefore an object of the present invention to provide an absorptive article which may be folded while keeping the user's hands away from the absorber, and may be handled in a hygienic manner.

### [Summary of Invention]

A first aspect of the present invention is summarized as an absorptive article (sanitary napkin 101) which has a liquid-permeable top sheet (top sheet 102), a liquid-impermeable back sheet (back sheet 103), and an absorber (absorber 104) disposed between the top sheet and the back sheet, and capable of absorbing body fluid. The absorptive article has insertion holes positioned on both sides in the width-wise direction (width-wise direction W) of the absorptive article, and extended along the longitudinal direction (in the longitudinal direction L) from the front end side (front end side Fr) of the absorptive article towards the rear end side (rear end side Ba) of the absorptive article; and long members (long members 106) inserted through the insertion holes. Each insertion hole has an opening (opening 108) positioned on the front end side of the absorptive article; and a fixing portion (fixing portion 112) positioned on the rear end side of the absorptive article. Each long member has a long portion (long portion 111) inserted through each insertion hole; and a draw-out portion (draw-out portion 109) continued from each long portion and drawn through each opening into the outside of each insertion hole. The rear end of the long portion positioned on the rear end side of the absorptive article is fixed to the fixing portion.

In the present invention, the long portions of the long members are provided on both sides in the width-wise direction of the absorptive article, and the rear ends of the long portions are fixed. As a consequence, if the draw-out portions of the long members inserted through the insertion holes are pulled by the user's hand, the absorptive article may be rolled up while keeping the top sheet inside and the back sheet outside. Because the rolling-up may be accomplished while keeping the top sheet inside, the user can handle the absorptive article in a hygienic manner, without bringing her hand into contact with the absorber.

### [Brief Description of Drawings]

FIG. 1 is an expanded view illustrating a sanitary napkin according to embodiment 1-1 of the present invention;
FIG. 2 is a sectional view taken along line A1-A1 in FIG. 1;
FIG. 3 is a perspective view showing a form of disposal;
FIG. 4 is an expanded view of a sanitary napkin according to another mode of embodiment 1-1 of the present invention;
FIG. 5 is a sectional view illustrating still another mode of embodiment 1-1 of the present invention;
FIG. 6 is an expanded view illustrating a sanitary napkin according to embodiment 2-1 of the present invention;
FIG. 7 is a sectional view taken along line M2-M2 in FIG. 6; and
FIG. 8 includes plan views illustrating procedures of unit-of-use packaging.

### [Detailed Description of The Preferred Embodiments]

### <First Embodiment>

### (Embodiment 1-1)

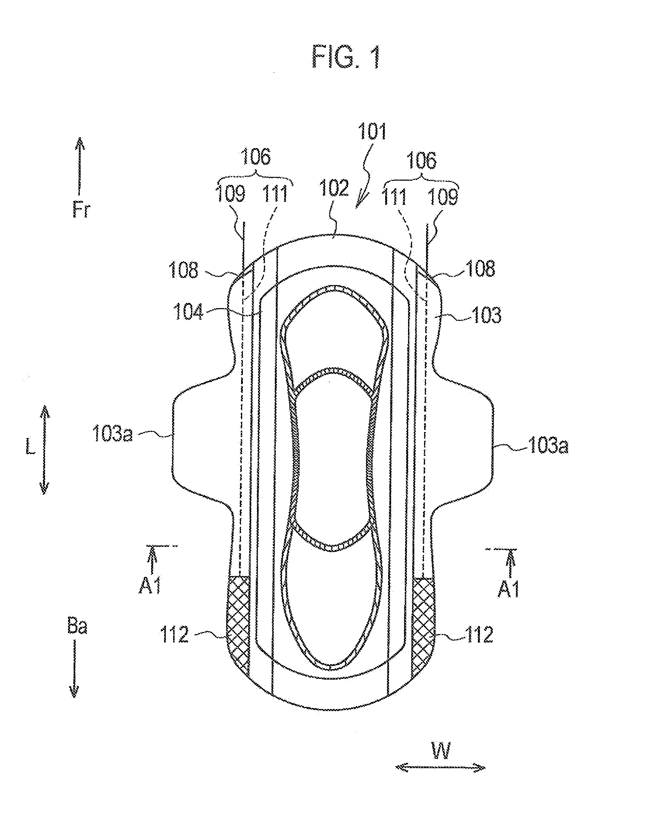

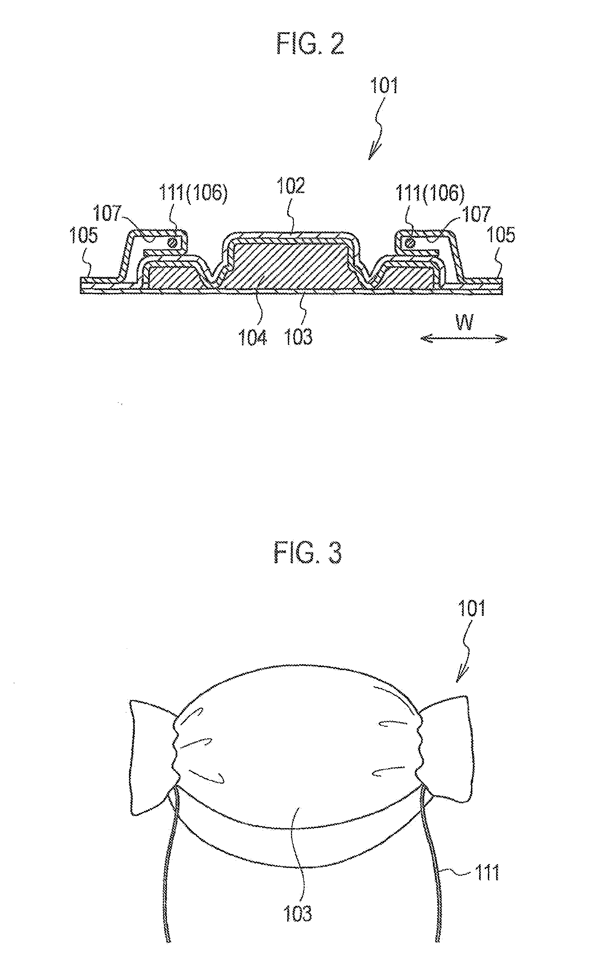

FIG. 1 an expanded view illustrating the sanitary napkin 101 according to embodiment 1-1 of the present invention. FIG. 2 is a sectional view taken along line A1-A1 in FIG. 1. FIG. 3 is a perspective view showing a form of disposal. The absorptive article of this embodiment configures a sanitary napkin 101. The sanitary napkin 101 has the top sheet 102, the back sheet 103, the absorber 104 disposed between the top sheet 102 and the back sheet 103, the side-sheets 105, and the long members 106.

The top sheet 102 is disposed on the skin contact surface side. The top sheet 102 is formed using a liquid-permeable material. Perforated or non-perforated, non-woven fabric, porous plastic sheet and so forth may be adoptable to the top sheet 102. The top sheet 102 is locally varied in the basis weight or density, after being subjected typically to heat embossing from the skin contact surface side, in a laterally symmetrical manner.

More specifically, portions different in the basis weight or density are alternately formed, along the longitudinal direction L from the front end side Fr of the sanitary napkin 101 towards the rear end side Ba of the sanitary napkin 101.

The absorber 104 is aimed at absorbing body fluid, such as menstrual blood passed through the top sheet 102. The absorber 104 is formed using fluffed pulp, air-raid, non-woven fabric, and a super absorbent polymer.

Chemical pulp, cellulose fiber, or artificial cellulose fibers such as rayon and acetate may be adoptable as the fluffed pulp.

As the super absorbent polymer, particulate or fibrous polymers of starch-base, acrylate-base and amino acid-base may be adoptable.

The air-raid, non-woven fabric is a non-woven fabric obtained by allowing pulp and synthetic fiber to bond thermally, or to bind with each other using a binder. As the air-raid, non-woven fabric, spunlace fabric, spunbond fabric, thermal bond fabric, melt-blown fabric, needle punched fabric and air-through fabric may be adoptable. Synthetic fibers including olefin-base ones such as polyethylene and polypropylene, polyester-base and polyamide-base may be adoptable as source fibers composing the non-woven fabric. Besides these materials, regenerated fibers such as rayon and cupra, and natural fibers such as cotton are also adoptable.

The back sheet 103 serves as a surface opposed to, and brought into contact with clothes such as shorts. The back sheet 103 is formed using a liquid-permeable material. As the back sheet 103, resin films including those composed of polyolefin such as polyethylene and polypropylene; polystyrene, polyurethane and so forth may be adoptable. Porous (moisture-permeable) polyolefin sheets having inorganic particles such as calcium carbonate, barium sulfate and so forth dispersed therein, stacked non-woven fabric manufactured by spun-bond method/melt-blown method/spun-bond method (denoted as SMS non-woven fabric, hereinafter), and stacked non-woven fabric manufactured by spun-bond method/melt-blown method/ melt-blown method/ spun-bond method (denoted as SMMS non-woven fabric, hereinafter) may be adoptable. These materials may be used alone, or in a mixed manner, or in a stacked manner. In the midway portion in the longitudinal direction L of the back sheet 103, there are provided a pair of wings 103a while being laterally spread. The wings 103a are provided with a pressure-sensitive adhesive as a binding member. By the contribution of the pressure-sensitive adhesive, the sanitary napkin 101 may be attached to the clothes.

The side-sheets 105 are bonded to at least a part of both sides of the top sheet 102. More specifically, the side-sheets 105 are disposed on both sides in the width-wise direction W of the absorber 104, along the longitudinal direction L. The side-sheets 105 are partially adhered onto the top sheet 102. The side-sheets 105 rise up from the top sheet 102, when the sanitary napkin 101 is used. Accordingly, the side-sheets 105 may act as preventing side leakage of body fluid. As the side-sheets 105, a hydrophobic or water-repellent non-woven fabric may be adoptable. In this case, the side-sheets 105 may be provided with the elastic members disposed in a stretched manner. In this manner, the rise-up state from the top sheet 102 may be kept in a reliable manner.

There are also provided insertion holes 107 positioned on both sides in the width-wise direction W of the sanitary napkin 101, and linearly extended along the longitudinal direction L from the front end side Fr of the sanitary napkin 101 towards the rear end side Ba of the sanitary napkin 101. The insertion holes 107 are formed by the top sheet 102 and the side-sheets 105.

An opening 108 is formed to each insertion hole 107 extended in the longitudinal direction L, so as to be positioned on the front end side Fr of the sanitary napkin 101. The insertion holes 107 in this case are provided in both side portions in the width-wise direction of the absorber 104.

There is also provided a fixing portion 112 to each insertion hole 107, while being positioned on the rear end side Ba of the sanitary napkin 101.

In this embodiment, the insertion holes 107 are provided between the side-sheets 105 and the top sheet 102. Alternatively, the insertion holes 107 may be provided between the top sheet 102 and the back sheet 103, and on both sides in the width-wise direction W of the absorber 4. This structure is meritful in terms of facilitating formation of the insertion holes 107.

Alternatively, for the case where the side-sheets 105 are folded on the absorber 104 side as illustrated in FIG. 2, the insertion holes 107 may be disposed so as to be held between the upper and lower side-sheets 105.

The long members 106 are inserted through the insertion holes 107. Each long member 106 has a long portion 111 inserted through each insertion hole 107, and a draw-out portion 109 continued from each long portion 111 and drawn through each opening 108 into the outside of each insertion holes 107. The long portions 111 are disposed on both sides in the width-wise direction W of the sanitary napkin 101, along the longitudinal direction L. Each long portion 111 is inserted in each insertion hole 107. The rear end side (bottom side in FIG. 1) in the longitudinal direction of each long portion 111 is fixed to the sanitary napkin 101. On the other hand, the front end side in the longitudinal direction of each long portion 111 is drawn out from each opening 108 of each insertion hole 107, and is given as the draw-out portion 109.

The long members 106 may be configured by a non-stretchable string component having a diameter of 1 to 7 mm, and preferably 2 to 5 mm. If the diameter of the long members 106 is smaller than 1 mm, the long members 106 may bite into the fingers and may hurt them, in the process of pulling the long members 106. If the diameter of the long members 106 exceeds 7 mm, the wearability may be degraded because the long members 106 may occupy a most area of the insertion holes 107. For the case where string components are used for the long members 106, the string components may be hydrophobidized, and may preferably be subjected to water-repellent finish, so as to prevent menstrual blood from being guided. Besides synthetic fibers including olefin-base ones such as polyethylene and polypropylene, and those of polyester-base and polyamide-base, regenerated fibers such as rayon and cupra, and natural fibers such as cotton are also adoptable as source fibers composing the long portions 111.

With respect to the individual long portions 111 of the long members 106, the length of the draw-out portions 109 drawn out from the openings 108 of the insertion holes 107 is set to 30 to 200 mm, and preferably set to 40 to 150 mm. If the length of the draw-out portions 109 is shorter than 30 mm, the long portions 111 may remain in the shorts or in the sanitary napkin 101, so that the long portions 111 may be stained with menstrual blood and may become unhygienic. What is worse, the long portions 111 drawn out through the openings 108 into the outside of the insertion holes 107 may be difficult to pinch when the used sanitary napkin 101 is disposed. If the length of the draw-out portions 109 exceeds 200 mm, the draw-out portions 109 may entangle outside the sanitary napkin 101, and may be unavailable at the time of disposal.

The fixing portions 112, illustrated in FIG. 1, fix the rear end side of the long portions 111. The fixing portions 112 may be formed by heat embossing or by bonding using a hotmelt adhesive (HMA), effected from the skin contact surface side of the side-sheets 105. For the case where heat embossing is adopted, the area to be embossed may be determined by the length in the width-wise direction W set to as long as the thickness of the long portions 111 added by a length of not longer than 5 mm, and by the length in the longitudinal direction L set to as long as 1 to 10 mm. The fixing portions 112 are provided 1 to 10 mm recessed from the edges of the sanitary napkin 101.

The strength of fixation of the long portions 111 with respect to the fixing portions 112 may be set larger than the rigidity in the longitudinal direction L of the sanitary napkin 101. The taper rigidity of the sanitary napkin 101 in the longitudinal direction L is set to the range from 50 to 90 mg, and preferably to the range from 60 to 80 mg. If the taper rigidity is smaller than 50 mg, the sanitary napkin 101 *per se* may unnecessarily be softened, and the sanitary napkin 101 may be twisted during use. If the taper rigidity exceeds 90 mg, the sanitary napkin 101 may be too hard to fit the body, and may degrade the comfortableness of use. For these reasons, the strength of fixation of the long portions 111 with respect to the fixing portions 112 is set to 100 mg or around.

By pinching the draw-out portions 109 of the left and right long members 106, and pulling the long portions 111 through the openings 108 into the outside of the insertion holes 107, the sanitary napkin 101 may be rolled up while keeping the top sheet 102 and the absorber 104 inside, and the back sheet 103 outside, as illustrated in FIG. 3.

This is partially ascribable to that the sanitary napkin 101 is used while keeping the surface of contact with the user, composed of the top sheet 102 and the absorber 104, curved so as to embrace the body. This is ascribable also to that, since the top sheet 102 side of the absorber 104 is locally varied in the basis weight or density, when compared between the top sheet 102 side of the absorber 104 and the back sheet 103 side of the absorber 104, so that the sanitary napkin 101 may be rolled up, while being originated from the points where the basis weight or density vary.

More specifically, portions varied in the basis weight or density are alternately arranged, along the longitudinal direction L from the front end side Fr of the sanitary napkin 101 towards the rear end side Ba of the sanitary napkin 101, so that the portions smaller in the basis weight or density may serve as the origins.

In addition, since the portions having smaller basis weight or density are formed in a laterally symmetrical manner, so that the sanitary napkin 101 will easily produce lines of folding in the width-wise direction W. Accordingly, the sanitary napkin 101 may be rolled up so as to originate from these lines of folding, while keeping the top sheet 102 and the absorber 104 inside.

Since the sanitary napkin 101 may be rolled up while keeping the top sheet 102 and absorber 104 inside, the user can handle the napkin in a hygienic manner without touching menstrual blood adhered thereto. It may be also possible to confine the odor. Since the sanitary napkin 101 once rolled up does not recover the initial extended form, so that the rolled sanitary napkin 101 may be discarded as it is. In addition, in this embodiment, the sanitary napkin 101 may be varied in the curvature, by pulling the draw-out portions 109. By varying the curvature of the sanitary napkin 101, the napkin may be improved in the fitness to the body, and may reduce leakage of body fluid.

It may also recommendable to attach an adhesive tape or cotton fastener tape, to the individual draw-out portions 109 of the long members 106. Since the draw-out portions 109 may temporarily be fixed to appropriate portions of the sanitary napkin 101 in this way, the appearance of the sanitary napkin 101 may be improved.

### (Other Embodiment 1)

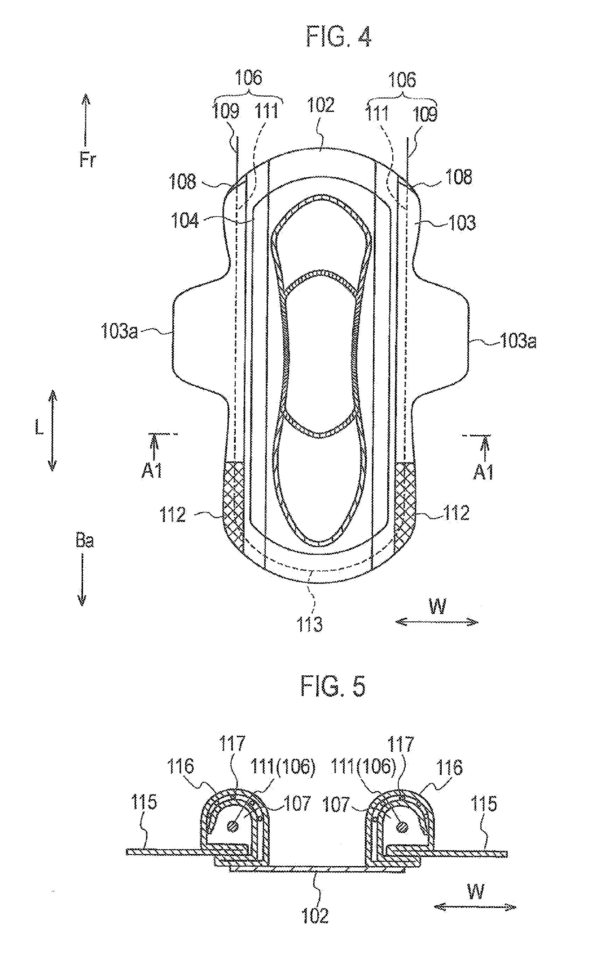

FIG. 4 illustrates another embodiment according to embodiment 1-1 of the present invention. Note that any identical components in the above-described embodiment will be given with the same reference numerals.

In this embodiment, the long members 106 are formed with a single string. The string is turned around on the rear end side Ba of the sanitary napkin 101, and respectively drawn out through the openings on the front end side Fr of the sanitary napkin 101. The long members 106 composed of a single string are turned around on the rear end side in the longitudinal direction L (turnaround portion 113). The long members 106 are straightly extended from both side portions in the width-wise direction of the turnaround portion 113, along the longitudinal direction. The long members 106 are fixed to the sanitary napkin 101 at the fixing portions 112 close to the turnaround portion 113. Both end portions of the string, which compose the individual front end portions of the long members 106 in the longitudinal direction L, are drawn out from the openings 108 of the insertion holes 107. Similarly to as described in the above embodiment, the sanitary napkin 101 may be rolled up by pulling the drawn-out ends, while keeping the top sheet 102 and the absorber 104 inside, and the back sheet 103 outside.

### (Other Embodiment 2)

FIG. 5 illustrates still another embodiment according to embodiment 1-1 of the present invention. Note that any identical components in the above-described embodiment will be given with the same reference numerals.

In this embodiment, the insertion holes 107 are configured using the inner spaces of gathered portions 116 composed of the side-sheet 115 and elastic members 117 disposed thereto. By using the inner spaces of the gathered portions 116 as the insertion holes 107, it may be no more necessary to provide the insertion holes 107, allowing therethrough insertion of the long members 106, to the sanitary napkin 101.

In this embodiment, the openings 108 of the insertion holes 107 and the fixing portions 112 are formed on the front end side Fr and the rear end side Ba of the sanitary napkin 101, respectively. The openings 108 of the insertion holes 107 and the fixing portions 112 may be also formed on the rear end side Ba and the front end side of the sanitary napkin 101, respectively. In this case, the front end of the long members 106 on the front end side Fr is fixed on the fixing portions 112 and drawn through the openings 108 to the outside of the insertion holes 107, to thereby give the draw-out portions 109.

This application is based upon and claims the benefit of priority from the prior Japanese Patent Application No. JP 2008-093283, filed on 31 March, 2008; and Japanese Patent Application No. JP 2009-030324, filed on 12 February, 2009, the entire contents of which are incorporated herein by reference.

### <Second Embodiment>

### [Background Art]

Patent Literature 2 discloses a sanitary napkin, having inner compressed trenches formed while being extended in the longitudinal direction on the left-and-right sides of an absorber, and outer compressed trenches formed outside of the inner compressed trenches while being spaced therefrom and extended in the longitudinal direction.

Patent Literature 3 discloses a sanitary napkin, having side leakage barrier trenches formed on the left-and-right sides of an absorber while being extended in the longitudinal direction; coupled leakage barrier trenches coupling the side leakage barrier trenches, formed to the front and the rear of the absorber; and leakage barrier walls having elastic members, and provided on the left-and-right sides of the absorber. The sanitary napkin is designed to be folded at a folding portion located where stretchability ascribable to elastic members is not available, for the convenience of unit-of-use packaging.

Patent Literature 4 discloses a sanitary napkin, having leakage barrier trenches provided on the left-and-right sides of an absorber, and having leakage barrier walls outside the leakage barrier trenches, wherein the leakage barrier walls are provided with elastic members extended in a stretched manner in the longitudinal direction.

Patent Literature 2: Japanese Patent Application No. JP 2004-181084
Patent Literature 3: Japanese Examined Patent No. JP 3824452
Patent Literature 4: Japanese Patent Application No. JP 2007-89906

### [Disclosure of the Invention]

The sanitary napkin disclosed in Patent Literature 2 has a geometry such that the inner compressed trenches and the outer compressed trenches are gradually widened over the region from the front center towards the mid center. As a consequence, all forces possibly applied by the femur during use of the sanitary napkin may be concentrated at a single point in the front center region, and thereby the absorber is raised up towards the vaginal opening of the female user. Accordingly, the blood discharged from the vaginal opening may more reliably be accepted. However, for the case where the sanitary napkin could not surely be attached to the clothes such as shorts, a gap may be produced between the absorber and the vaginal opening, because the absorber could be fitted to the vaginal opening only in a pinpoint manner. The gap raises a risk of leakage of blood.

The sanitary napkin disclosed in Patent Literature 3 may impair the barrier blocking effect, if it is folded at portions of the leakage barrier walls where the stretching force is applied by the elastic members, since such way of folding may result in collapse of the leakage barrier walls. For this reason, the sanitary napkin needs be folded at the portions where the stretching force by the elastic members is not exerted. Taking a margin for the folding into account, the portion contributive to the leakage barrier effect needs be reduced. The leakage barrier effect may consequently be degraded, and thereby a problem of blood leakage may arise.

If the stretching force of the elastic members is increased in the sanitary napkin disclosed in Patent Literature 4, in order to enhance the leakage barrier effect of the leakage barrier walls, the rigidity of the absorptive article may degrade. As a consequence, the leakage barrier walls can no more keep on rising, and the blood leakage may occur. If two leakage barrier trenches, such as the inner one and the outer one, are formed on each of the left-and-right sides of the absorber, aiming at enhancing the leakage barrier effect of the portion opposed to the excretory zone of the user, addition of one leakage barrier trench may reduce the area of the portion opposable to the excretory zone, and thereby the blood leakage may occur.

It is therefore an object of the present invention to provide an absorptive article capable of efficiently preventing blood leakage.

The present invention is summarized as an absorptive article (sanitary napkin 201) which includes a body fluid-permeable top sheet (top sheet 202), a body fluid-impermeable back sheet (back sheet 203), and an absorber (absorber 204) disposed between the top sheet and the back sheet, and has a pair of leakage barrier walls (leakage barrier wall 205) in both side portions in the width-wise direction of the absorber along the longitudinal direction, and having, between the pair of leakage barrier walls, leakage barrier trenches (leakage barrier trenches 208) provided on both sides of a skin contact portion of the absorber. The leakage barrier trenches have arch-wise projected portions in both side portions in the width-wise direction of the skin contact portion, and the absorber has compressed portions (compressed portions 215) having rigidity larger than that in the region including at least the skin contact portions of the leakage barrier trenches, in regions (regions 209) between the leakage barrier walls and the leakage barrier trenches.

According to the present invention, the compressed portions which have a rigidity larger than that in the region including at least the skin contact portions of the pair of leakage barrier trenches, are provided in the regions between the leakage barrier walls and the leakage barrier trenches. As a consequence, the leakage barrier walls may be less likely to collapse under body pressure during use of the absorptive article, and the leakage barrier walls may more readily rise up, so that leakage of blood may more reliably be prevented.

### [Detailed Description of The Preferred Embodiments]

### (Embodiment 2-1)

### The present invention will specifically be explained referring to embodiment 2-1.

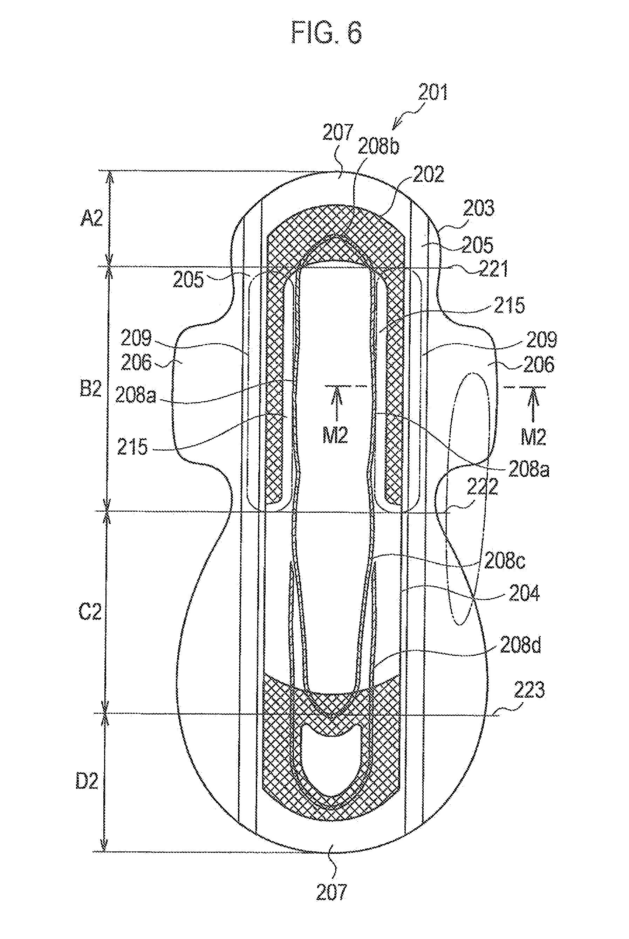

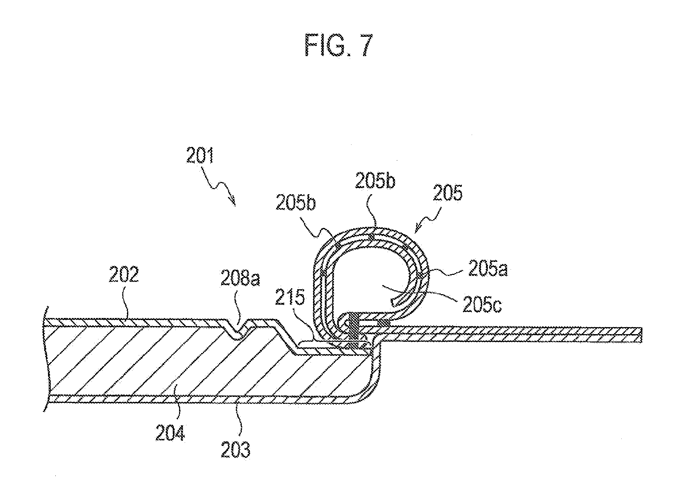

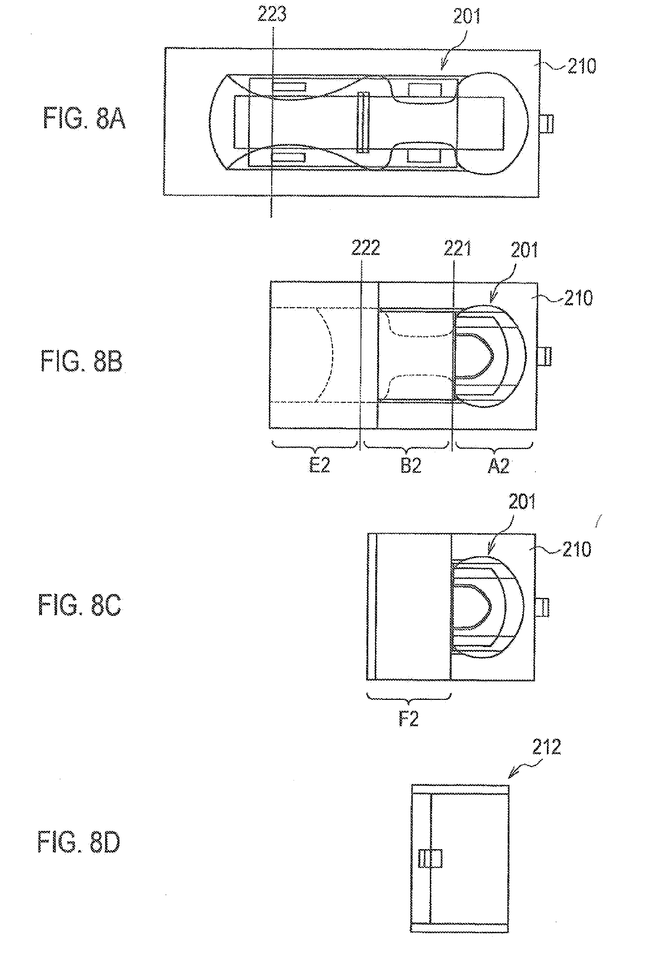

FIG. 6 is an expanded view illustrating a sanitary napkin according to an embodiment of the present invention. FIG. 7 is a sectional view of the sanitary napkin according to the embodiment of the present invention illustrated in FIG. 6, taken along line M2-M2. FIG. 8 includes plan views illustrating procedures of unit-of-use packaging according to the sanitary napkin of the embodiment of the present invention.

The absorptive article of the present invention configures the sanitary napkin (referred to as napkin, hereinafter) 201. The napkin 201 has an oblong form in the longitudinal direction, extended backward so as to cover a region close to the tailbone. As illustrated in FIG. 6 and FIG. 7, the napkin 201 configures the top sheet 202 as a liquid-permeable surface layer, the back sheet 203 as a liquid-impermeable back layer, and the absorber 204 as a liquid-retaining layer provided between the top sheet 202 and the back sheet 203.

Perforated or non-perforated, non-woven fabric, porous plastic sheet and so forth may be adoptable to the top sheet 202.

Hydrophobic non-woven fabric, water-impermeable plastic film, laminated sheet of non-woven fabric and water-impermeable plastic sheet and so forth may be adoptable to the back sheet 203. Besides these materials, also SMS non-woven fabric obtained by stacking melt-blown non-woven fabrics excellent in water-proofness, and a spunbond, non-woven fabric excellent in strength, may be adoptable.

As the absorber 204, fluffed pulp, air-raid, non-woven fabric, and a super absorbent polymer may be adoptable. Chemical pulp, cellulose fiber, or artificial cellulose fibers such as rayon and acetate may be adoptable as the fluffed pulp. As the air-raid, non-woven fabric, non-woven fabric obtained by allowing pulp and synthetic fiber to bond thermally or to bind with each other using a binder, may be selectable. As the super absorbent polymer, particulate or fibrous polymers of starch-base, acrylate-base and amino acid-base may be adoptable. As the non-woven fabric, spunlace fabric, spunbond fabric, thermal bond fabric, melt-blown fabric, needle punched fabric and air-through fabric may be adoptable as the air-raid, non-woven fabric. As a source fiber composing the non-woven fabric, not only synthetic fibers including olefin-base ones such as polyethylene and polypropylene, polyester-base, polyamide-base and polyamide-base, but also regenerated fibers such as rayon and cupra, and natural fibers such as cotton are adoptable.

In the left-and-right side portions on the top sheet 202 side of the napkin 201, in other words, both side portions in the width-wise direction of the absorber 204, there are disposed a pair of leakage barrier walls 205 along the longitudinal direction. The individual leakage barrier walls 205 rise up from portions close to the left-and-right side edges of the absorber 204.

The napkin 201 may be divided into a front portion A2 of the skin contact portion, a skin contact portion B2, a first rear portion C2, and a second rear portion D2, positioned in this order when viewed in the direction from the belly side (front) towards the back side (rear). The skin contact portion B2 is a portion opposed to the excretory zone of the user who wears the napkin 201. The front portion A2 is located in adjacent to the skin contact portion B2, and located on the front of the napkin 201. The first rear portion C2 and the second rear portion D2 are located on the rear of the skin contact portion B2. These portions are bounded by folding portions 221, 222 and 223. The front portion A2 and the skin contact portion B2 are bounded by the folding portion 221. The skin contact portion B2 and the first rear portion C2 are bounded by the folding portion 222. The first rear portion C2 and the second rear portion D2 are bounded by the folding portion 223. The skin contact portion B2 serves as a skin contact portion brought into contact with the user's skin.

The back sheet 203 is geometrically swelled out laterally in the width-wise direction from the side edges of the absorber 204. In the skin contact portion B2 of the swelled napkin 201, the back sheet 203 forms a pair of wings 206. Although not illustrated, the back sheet 203 and the wings 206 have, on the surface thereof opposed to the clothes, a tacky layer formed thereon, for the convenience of fixation of the napkin 201 to the clothes. The tacky layer is protected by a release paper not illustrated. The top sheet 202 and the back sheet 203 are respectively swelled out from the front and rear edges of the absorber 204. The swelled top sheet 202 and the back sheet 203 are bonded and fixed to each other, to thereby form end seal portions 207.

The leakage barrier walls 205 are disposed in the left-and-right side potions of the absorber 204. Each leakage barrier wall 205 is composed of a band-form sheet 205a and a plurality of elastic members 205b (see FIG. 7). At the fixed end of each leakage barrier wall 205, a portion of the leakage barrier wall 205 is bonded to the top sheet 202. At a position slightly outward from the side edge of the absorber 204, a part of each leakage barrier wall 205 rises up from the root of each wing 206. The leakage barrier wall 205 in this embodiment is configured to form a hollow portion 205c. The leakage barrier wall 205, configured to form the hollow portion 205c, may more readily rise up from the back sheet 203. The leakage barrier wall 205 may have a geometry having a base wall portion, such as Σ-shape and T-shape. As the band-form sheet 205a, non-woven fabric may be adoptable.

The elastic member 205b are fixed while being stretched, in the band-form sheet 205a composing the leakage barrier wall 205. Heat seal, hotmelt adhesive and so forth may be adoptable as a means for fixing the elastic members 205b.
The elastic members 205b are not specifically limited, so far as they may be adoptable to the absorptive article, such as porous materials of polyolefins and polyurethanes, and natural rubber. The elastic members 205b may have a form of string, band, film and so forth. In this embodiment, a string having a thickness of 470 dtex is used.

Six or more yarns of elastic members 205b are disposed on one side of the absorber 204. The elongation factor of the individual elastic member 205b is set at high values, and almost equally for all yarns. In this embodiment, the individual elastic members 205b are stretched at an elongation factor of 1.4 or larger. The elongation factor is set constant, irrespective of geometry of the leakage barrier walls 205.

On the top sheet 202 side and in both side portions of the napkin 201, the individual leakage barrier trenches 208 are respectively formed so as to extend in the longitudinal direction of the napkin 201. Each leakage barrier trench 208 is formed by compression using a compressive means, such as embossing, of the top sheet 202 and the absorber 204, effected from the top sheet 202 side. Each leakage barrier trench 208 is compressed and integrated. The individual leakage barrier walls 205 are respectively located outside the individual leakage barrier trenches 208. Each leakage barrier trench 208 has a geometry almost symmetrical about the center line in the longitudinal direction of the napkin 201. The individual leakage barrier trenches 208 are linked with each other at the front and rear ends, and thereby the leakage barrier trenches 208 are given in a closed pattern as a whole.

The portions of the individual leakage barrier trenches 208, fallen in the skin contact portion B2, are given as center curved trenches 208a curved in an outwardly convex pattern in the width-wise direction of the napkin 201. The center curved trenches 208a are geometrically swelled outwardly in the width-wise direction, in both side portions in the skin contact portion B2. The portions in the front and rear of the center curved trenches 208a extend in the longitudinal direction of the napkin 201, to thereby form a front trench 208b and a rear trench 208c, respectively. The leakage barrier trench 208, composed of the center curved trenches 208a, and the front trench 208b and the rear trench 208c extended from the front and rear thereof, is extended almost over the entire length of the napkin 201. From the rear trench 208c, a rear trench 208d is formed so as to further extend therebehind in the longitudinal direction. The rear trench 208d has a U-shape. The rear trench 208d vertically extends across the first rear portion C2 and the second rear portion D2. The front trench 208b and the rear trench 208d are curved in a outwardly convex pattern in the width-wise direction of the napkin 201, similarly to the center curved trenches 208a.

In this embodiment, compressed portions 215 are provided in the regions 209 indicated by a dashed line, which does not overlap the center curved trenches 208a, on the outer side in the width-wise direction of the center curved trenches 208a located in the contact portion, and does not overlap the folding portion 221 in the longitudinal direction. The compressed portions 215 are compacted by subjecting the absorber 204 to a compressive means such as embossing. The compressed portions 215 are given as a portion high in the rigidity. Because the elastic member 205b composing the leakage barrier walls 205 are elongated at a large elongation factor, the skin contact portion B2 may possibly collapse in the geometry by the contribution of the force of shrinkage of the elastic members 205b. By forming the compressed portions 215 in the regions 209, the skin contact portion B2 may be prevented from collapsing in the geometry, while keeping readiness in mountain-folding originated at the center curved trench 208a unchanged. Accordingly, the absorption surface, or the surface on the top sheet 202 side, may stably rise up to closely contact with the user's body. By the close contact of the absorption surface with the user's body, side leakage of blood may exactly be avoidable.

By virtue of thus-configured compressed portions 215, it may be no more necessary to additionally provide the leakage barrier trenches outside, in the width-wise direction, of the center curved trenches 208a, unlike the conventional cases. As a consequence, there may be no fear of increasing the region hardened by double leakage barrier trenches, and thereby the user may be prevented from feeling uncomfortableness about the hardness during use.

In the process of manufacturing the sanitary napkin, the top sheet 202 and the leakage barrier walls 205 are integrated, and then leakage barrier trench 208 (hinge) is formed by embossing, for the purpose of bonding with the absorber 204. If the distance between the leakage barrier trench 208 and the leakage barrier walls 205 is too small, also the leakage barrier walls 205 may undesirably be entrained and embossed together. For this reason, at least 5 mm or around is necessary for the distance between the leakage barrier trenches 208 and the leakage barrier walls 205. In order to cover the crotch of a female user, which is generally 35 to 45 mm wide, the width of the absorber 204 between the leakage barrier walls 205 is designed to 55 to 65 mm, taking dislocation during use into consideration. As described in the above, if the additional leakage barrier trenches are provided outside in the width-wise direction of the center curved trenches 208a, the distance between the center curved trenches 208a may correspondingly be narrowed, only to ensure a small area for the absorbing surface. In contrast, in the compressed portions 215 in this embodiment, the absorber 204 is compacted by a compressive means such as embossing. By virtue of the compaction, the napkin 201 may be improved in the fitness, without causing uncomfortableness to the user. Accordingly, the leakage barrier effect further increases. In particular, the risen-up portion of the absorber 204 is surrounded by the leakage barrier trench 208 having a closed pattern, the stability of the planar geometry may further be improved.

In order to more stably rise up the absorption surface, it may be effective to increase the basis weight (density) of the absorber 204 placed between the leakage barrier trenches 208. In this case, if the basis weight of the absorber 204 placed between the leakage barrier trenches 208 is adjusted to 300 to 1500 g/m², and in particular 500 to 1200 g/m², the absorption surface may be risen up in a more stable manner. The range of basis weight described in the above may be advantageous also in that the napkin 201 may be more readily pushed upwardly to the body, by pressure laterally applied to the napkin 201, assuming the center curved trenches 208a as the origin of folding. The main effect expectable from the range of basis weight of 300 to 900 g/m² may be such that the center curved trenches 208a may serve as the origin of folding. In the range of basis weight from 900 to 1500 g/m², in addition to the effect that the center curved trenches 208a may serve as the origins of folding, also another effect may be expectable in that the mid-to-high portions may more readily rise up while slightly deforming themselves into a drum shape.

The basis weight (density) of the portion of the absorber 204 fallen outside the leakage barrier trench 208 is preferably 100 to 400 g/m², and particularly preferably 150 to 300 g/m². By adjusting the basis weight of the portion of the absorber 204 fallen outside the leakage barrier trench 208 to the above-described range, the napkin 201 may comfortably be worn by the user, without causing uncomfortableness of hardness ascribable to compaction, during the use. The configuration is preferable also from the viewpoints that the absorption surface may more stably be raised up, and that the napkin 201 may more readily be pushed up towards the body, assuming the center curved trenches 208a as the origin of folding.

The regions 209 may preferably have a Gurley rigidity value of preferably 1000 to 2000 mg, and particularly 1200 to 1800 mg, in view of preventing the user from feeling uncomfortableness of hardness during use, and of keeping a desirable rigidity of the napkin 201. The portion of the absorber 204 fallen between the leakage barrier trenches 208, not compacted by a compressive means such as embossing, has a large basis weight (density). In view of avoiding twist of the napkin 201 during use, and keeping comfortableness of use, the Gurley rigidity value may preferably be adjusted to 150 to 800 mg, and particularly 300 to 500 mg.

Further from the similar point of view, the center curved trenches 208a fallen in the skin contact portion B2 may preferably be 50 to 120 mm long in the longitudinal direction, and particularly 70 to 100 mm long. Since the region from the perineum to the anus causes a large change in the curvature of body, so that leakage obliquely backward may be likely to occur. The regions of the rear trench 208c and the rear trench 208d fallen in the first rear portion C2 may preferably be 50 to 120 mm long, in view of making a reliable fitting of the absorber 204 to the region from the perineum to the anus. The rear trench 208c and the rear trench 208d may particularly preferably be 70 to 100 mm long. The portions of the front trench 208b fallen in the front portion A2, and the rear trench 208d fallen in the second rear portion D2, may preferably be 20 to 70 mm long in the longitudinal direction, and particularly be 30 to 50 mm long.

In this manner, the napkin 201 may be given as a self-supporting product, while keeping a fitting performance in a plane geometry, without being affected by the force of shrinkage of the leakage barrier walls 205 stretched at a large factor of elongation.

### (Unit-of-Use Packaging Structure of Napkin)

The unit-of-use packaging structure of the napkin 201 will be explained.

The compressed portions of the napkin 201 are continuously or intermittently formed between the folding portion, where the absorptive article is folded for unit-of-use packaging at the center, and the front end of the absorber, in the longitudinal direction.

As illustrated in FIG. 8, the napkin 201 is folded three times in the longitudinal direction, together with a packaging sheet 210. The packaging sheet 210 may be a non-woven fabric, film, or the like, but may not specifically be limited so far as it is generally applicable to the absorptive article. Those adapted to releasing may be preferable.

When the napkin 201 is folded, in the state illustrated in FIG. 8A, the second rear portion D2 is folded at the folding portion 223 so as to overlap the first rear portion C2, to thereby form region E2 (FIG. 8B). Next, the region E2 is folded at the folding portion 222 so as to overlap the skin contact portion B2, to thereby form region F2 (FIG. 8C). The region F2 is then folded at the folding portion 221, to thereby form a unit-of-use package 212 illustrated in FIG. 8D. In the process of folding, the regions 209 do not overlap the folding portion 222 as described in the above, instead only a single leakage barrier trench 208 on each of left-and-right side resides across the folding portion 222. Accordingly, the napkin 201 may be low in the rigidity, and more ready to be folded. In view of ensuring the self-supporting property, the front and rear fixing portions of the leakage barrier walls 205 and skin contact portion (region 209) essentially need be raised in the rigidity, through compaction by a compressive means such as embossing effected to the absorber 204. The folding portion 221 and the folding portion 222 of skin contact portion B2 are preferably not compacted. This is because the napkin 201 may be more difficult to be folded, if the folding portion 221 and the folding portion 222 in the contact portion B2, having a larger basis weight (density) than the second rear portion D2, is compacted by a compressive means such as embossing. The front portion A2 overlaps the region F2 to provide a unit-of-use package as illustrated in FIG. 8. Note that the region, over which the force of shrinkage of the elastic members 205b exerts, may be the folding portions 221, 222 and 223, and may be any region over the front end to the rear end of the napkin 201, without special limitation.

### (Operations and Effects)

In embodiment 2-1, since the compressed portions 215, having a larger rigidity than in the region containing the skin contact portion B2 of the leakage barrier trenches 208, are provided in the regions 209 between the leakage barrier walls 205 and the leakage barrier trenches 208, the napkin 201 may be less likely to collapse by the body pressure during use, and may more reliably prevent the leakage of blood, by the contribution of improved self-supporting property of the leakage barrier walls 205.

The inside portion of the center curved trench 208a, having a smaller rigidity, may cause mountain-folding while assuming the center curved trenches 208a as the origin of folding, when applied with the external force such as body pressure, and may come into close contact with the body. The compressed portions 215 outside of the center curved trenches 208a, having a larger rigidity, may be less likely to collapse even applied with the external force. Since the leakage barrier walls 205 remain uncollapsed after the package is opened, so that they may be less likely to collapse under body pressure. The leakage barrier walls 205 may be improved in the self-supporting property, by the contribution of a large force of shrinkage of the elastic member 205b. Accordingly, the side leakage preventive effect may be enhanced.

This application is based upon and claims the benefit of priority from the prior Japanese Patent Application No. JP2008-051073, filed on 29 February, 2008; the entire contents of which are incorporated herein by reference.

### [Industrial Applicability]

As has been described in the above, the absorptive article of the present invention may be handled in a hygienic manner, while keeping the hands away from the absorber, and may therefore be useful as the absorptive article for sanitary use.

The absorptive article of the present invention may effectively prevent also leakage of blood, and may therefore be useful as the absorptive article such as sanitary napkin.

## Claims

1. An absorptive article comprising a liquid-permeable top sheet, a liquid-impermeable back sheet, and an absorber disposed between the top sheet and the back sheet, and capable of absorbing body fluid, the absorptive article having:
insertion holes positioned on both sides in the width-wise direction of the absorptive article, and extended along the longitudinal direction from the front end side of the absorptive article towards the rear end side of the absorptive article; and
long members inserted through the insertion holes, wherein
each insertion hole has:
an opening positioned on the front end side of the absorptive article; and
a fixing portion positioned on the rear end side of the absorptive article;
each long member has:
a long portion inserted through each insertion hole; and
a draw-out portion continued from each long portion and drawn through each opening into the outside of each insertion hole; and
the rear end of the long portion positioned on the rear end side of the absorptive article is fixed to the fixing portion.

2. The absorptive article according to claim 1, wherein
the insertion holes are formed on both sides in the width-wise direction of the absorber, between the top sheet and the back sheet.

3. The absorptive article according to claim 1, wherein
the top sheet has, at least in portions on the left-and-right sides thereof, side-sheets bonded thereto, and
the insertion holes are provided between the side-sheets and the back sheet.

4. The absorptive article according to claim 1, wherein
the long member is formed by a single string,
the string being turned around on the rear end side of the absorptive article, and
both ends of the string are respectively drawn out from the openings on the front end side of the absorptive article.

5. An absorptive article comprising a liquid-permeable top sheet, a liquid-impermeable back sheet, and an absorber disposed between the top sheet and the back sheet, and capable of absorbing body fluid, the absorptive article having
insertion holes positioned on both sides in the width-wise direction of the absorptive article, and extended along the longitudinal direction from the front end side of the absorptive article towards the rear end side of the absorptive article; and
long members inserted through the insertion holes, wherein
each insertion hole has:
an opening positioned on the front end side of the absorptive article; and
a fixing portion positioned on the rear end side of the absorptive article;
each long member has:
a long portion inserted through each insertion hole; and
a draw-out portion continued from each long portion and drawn through each opening into the outside of each insertion hole; and
the front end of the long portion positioned on the front end side of the absorptive article is fixed to the fixing portion.

6. An absorptive article comprising a body fluid-permeable top sheet, a body fluid-impermeable back sheet, and an absorber disposed between the top sheet and the back sheet, and having a pair of leakage barrier walls provided on both sides in the width-wise direction of the absorber along the longitudinal direction, and having, between the pair of leakage barrier walls, leakage barrier trenches provided on both sides of a skin contact portion of the absorber, wherein
the leakage barrier trenches have arch-wise projected portions in both side portions in the width-wise direction of the skin contact portion, and
the absorber has compressed portions having rigidity larger than that in the region including at least skin contact portions of the leakage barrier trenches, in regions between the leakage barrier walls and the leakage barrier trenches.

7. The absorptive article according to claim 6, wherein
the compressed portions are embossed portions formed by compressing the absorber by embossing.

8. The absorptive article according to claim 6, wherein
the compressed portions are formed continuously or intermittently between a folding portion at the center of the absorptive article before being folded and packaged by unit of use, and the front end of the absorber.
